# EUROPEAN PATENT APPLICATION

(11) **EP 3 488 814 A1**
(43) Date of publication of application: **29.05.2019**
(21) Application number: 17802129.1
(22) Date of filing: 23.05.2017
(51) Int. Cl.: A61F 2/00

(54) **IMPLANTED URINARY CONTROL DEVICE**

(30) Priority: 25.05.2016 CN 201610356695
(71) Applicant: Zhang, Xiujun, Shanghai 200433 (CN)
(72) Inventor: SUN, Yinghao, Shanghai 200433 (CN)
(74) Representative: Viering, Jentschura & Partner mbB Patent- und Rechtsanwälte
(86) International application number: PCT/CN2017/085441
(87) International publication number: WO 2017/202283

(57) **Abstract**

Provided is an implanted urinary control device, which comprises two tool handles (1). The tool handles (1) each comprise a connecting portion, a urinary tract actuator (1.3) and a handle actuator (1.1). The two tool handles (1) are movably connected to each other via the connecting portions. The two handle actuators (1.1) move relative to each other, thereby controlling an opening motion of a first urinary tract actuator (1.3) relative to a second urinary tract actuator (1.3). The urinary tract actuator (1.3) of one of the tool handles (1) is positioned in front of the urinary tract actuator (1.3) of the other one of the tool handles (1). The device further comprises tool handle (1) rotation angle limiting mechanisms (3) for limiting opening angles of the urinary tract actuators (1.3). The implanted urinary control device has a simple structure, induces less trauma, and provides an ideal urinary control effect.

## Description

### TECHNICAL FIELD

The present invention relates to the medical devices technical field, and particularly, relates to an implantable urination control device implanted within the penis and capable of autonomously controlling urination.

### BACKGROUND

Urinary leakage and even urinary incontinence, which can be attributable to traumas, abnormality of neurological functions, surgical complications, aging and the like, are the major causes that severely affect a patient's quality of life and even lead to some diseases such as urinary tract infection. In view of the above, various techniques have been developed at present to solve this problem, and the primary solutions fall into the following two categories: (1) surgical treatment; and (2) non-surgical treatment.

Here, with regard to surgical treatment, reference may be made to implantable artificial urinary tract sphincter techniques represented by the solutions set forth in patent documents EP2810616A1 and US20080146868A1. Such techniques usually involve a urinary tract restricting device, a switch device, a connecting device, etc. In practice, as it is difficult to idealize the loosening adjustment of the urinary tract restricting device during surgery operation, urinary tract restriction may be rendered excessively tight, thereby leading to dysuria. Moreover, if the urinary tract restricting device is rendered relatively loose, urinary leakage may still occur. The major problem with this method lies in complex structures, complicated surgical steps, and severe surgical trauma, which hampers the promotion and application of this technique.

With regard to the non-surgical solutions for urinary incontinence, they try to solve the problem by means of a urine collector, a penis outer clamp and the like, such as the solutions mentioned in patent documents CN201410771255.8 and US6981505B2, which are clamped outside the penis body and featured by a penis clamping structure, an opening-closing structure, a urinary tract pressing structure or a urine collecting structure. However, these solutions often tend to cause discomfort to the human body when clamped on the penis body, and if used for a prolonged period of time, they may lead to poor supply of blood to the penis. The most critical problem is that the shape of the penis of a male may change dramatically at different times, different temperatures, etc., such that it is extremely difficult to achieve ideal mounting and urinary control for similar clamping structures.

In conclusion, there is as yet no urination controlling tool which provides an ideal urinary control effect and is safe and convenient to use.

### SUMMARY

It is an object of the present invention to overcome the drawbacks existing in the prior art, and provide an implantable urination controlling tool which is simple and stable in structure, less in trauma induced by the implantation manner and ideal in urinary control. This tool not only well solves the problem of urinary incontinence; more importantly, no trace of it can be found from the body surface, and it can be used conveniently in daily life and is maintenance-free for life.

In order to achieve the above object, the present invention provides an implantable urinary control device. The implantable urinary control device comprises two tool handles, wherein each of the tool handles comprises a connecting portion, a urinary tract actuator and a handle actuator; the implantable urinary control device also comprises a fixing block for connecting with the connecting portions of the two tool handles, wherein the urinary tract actuator of one of the tool handles is positioned in front of the urinary tract actuator of the other tool handle; the implantable urinary control device further comprises tool handle rotation angle limiting mechanisms for limiting opening angles of the urinary tract actuators.

The two handle actuators move relative to each other, thereby controlling an opening or closing motion of the urinary tract actuator of one of the tool handles relative to the urinary tract actuator of the other tool handle.

Preferably, the connecting portions of the two tool handles are parallel to each other, and the tool handle rotation angle limiting mechanisms are disposed on the connecting portions.

The parallel arrangement has the advantage of low requirements for motion space, and thus can better satisfy the requirement of implantation within a penis for space.

Preferably, the connecting portion is in the form of a connecting shaft.

When construed narrowly, the connecting shaft expressed herein is specifically embodied as a round rod-shaped member whose two ends are connected respectively with the urinary tract actuator and the handle actuator.

When the two handle actuators are controlled to move relative to each other, the tool handles may be made to rotate at a certain angle along the axial direction of the connecting shafts, which, in turn, drives the urinary tract actuators at opposite ends of the connecting shafts to rotate at a certain angle, thereby achieving the motion of occluding or opening the urinary tract.

When the handle actuators are controlled to achieve the opening and closing of the urinary tract actuators, the rotation angle limiting mechanisms correspond to a urinary tract opening stable state, a urinary tract closing stable state or the two stable states. Such design is aimed at allowing a patient, when urinating, to conveniently change the urinary tract state merely by toggling the handles once. Take the situation in which the rotation angle limiting mechanisms correspond to a urinary tract closing stable state as an example. When the urinary tract actuators are in a closing state, the rotation angle limiting mechanisms maintain a closing stable state; however, when the urinary tract actuators are in an opening state, the tool handle rotation angle limiting mechanisms may produce forces for returning them to the closing state.

The tool handle rotation angle limiting mechanisms may also be various spacing mechanisms commonly used in the mechanical domain. For example, preferably, the tool handle rotation angle limiting mechanisms are spacing protrusions; the spacing protrusions are disposed on the connecting portions; when one of the tool handles rotates, its spacing protrusion abuts against the spacing protrusion of the other tool handle upon rotation, thereby preventing the tool handles from continuing to rotate.

Preferably, the tool handle rotation angle limiting mechanisms are spring sheets, permanent magnets or spacing protrusions.

Preferably, the tool handle rotation angle limiting mechanisms also comprise spacing protrusions; the spacing protrusions are disposed on the connecting portions; when one of the tool handles rotates, its spacing protrusion abuts against the spacing protrusion of the other tool handle upon rotation, thereby preventing the tool handles from continuing to rotate.

Explanation is made below by taking the situation in which the connecting portion is in the form of a connecting shaft as an example:

When the rotation angle limiting mechanisms are spring sheets, the stable state to which they correspond is a single one, i.e. that the spring sheets either correspond to a urinary tract closing stable state or correspond to a urinary tract opening stable state. When urination is required, the handles are controlled by hand so as to artificially change the stable state. When a patient releases his hands after urination, the tool handles will automatically return to the urinary tract closing stable state under the action of the elastic force exerted by the spring sheet. When urination is required, the handle actuators need to be continuously pinched by hand so as to achieve the opening of the urinary tract. As the spring sheet exerts a continuous elastic force on the connecting segments at this time, the urinary tract actuators will automatically return to the urinary tract closing state when a patient releases his hands after urination.

Whether the spring sheet corresponds to the urinary tract closing stable state or the opening stable state depends on the relative relationship between the two urinary tract actuators. When the handle actuators are pinched together, the connecting shafts are made to rotate inwards relative to each other, and the spring sheet stores energy. If the urinary tract actuators are in an opening state relative to each other at this time, the spring sheet initially corresponds to the urinary tract closing stable state; if the urinary tract actuators are in a clamping state relative to each other at this time, the spring sheet initially corresponds to the urinary tract opening stable state.

When the rotation angle limiting mechanisms are magnets, they can simultaneously achieve the urinary tract opening stable state and the urinary tract closing stable state. Such design is aimed at allowing a patient, when urinating, to conveniently shift the urinary tract state from the closing state to the urinary tract opening state so as to achieve the action of urination, merely by toggling the handle actuators once. When urination is over, all that is needed is to adjust again the handle actuators so as to shift the urinary tract actuators from the opening state to the closing state.

When the rotation angle limiting mechanisms are spacing protrusions, they are disposed on the connecting shaft of one of the tool handles, such that when the relative rotation of the two tool handles reaches a set angle, the spacing protrusion will come into contact with the spacing protrusion on the other tool handle so as to prevent the two tool handles from rotating, thereby playing a role in limiting the rotation angle. The two spacing protrusions are disposed on the two tool handles in a mutually corresponding manner. During design, the combination of such design elements as height, width and radian can be used to ensure that further rotation is restrained after the rotation angle reaches a set value.

The tool handle rotation angle limiting mechanisms are compressible springs, and the connecting shaft is provided with a spring contact surface. Two ends of the compressible spring come into contact with the spring contact surfaces of the two connecting shafts, respectively, and the fixing block is provided with grooves for avoiding the compressible spring from coming into contact with the spring contact surfaces.

Their connection relationships have the following several preferred modes:

A window is disposed in the middle portion of the fixing block, and the grooves are positioned on side walls of the window and are also in communication with communication holes of the connecting shafts. The spring contact surface on each of the connecting shafts is a clamping slot, and two ends of the compressible spring respectively pass through the grooves of the fixing block to be connected with the clamping slots on the connecting shafts in a clamping manner.

The grooves are positioned on two sides of the fixing block and are also in communication with the communication holes of the connecting shafts. The fixing block is provided centrally with a mounting hole for fixedly connecting with a pin member in the center of the spring sheet, and two ends of the spring sheet abut the connecting shafts of the two tool handles.

The compressible spring may be in the form of a sheet-like spring, a cylindrical coiled spring or a bellow-type tension spring, etc.

Preferably, the spring sheet is arched, and inner sides of the two ends of the spring sheet abut the connecting shafts of the two tool handles.

Alternatively, the spring sheet is planar. The connecting shafts of the tool handles extend out of the grooves to form connecting segments, and two ends of the planar spring sheet abut the connecting segments.

The tool handle rotation angle limiting mechanisms is composed of a permanent magnet fixed on the fixing block and a permanent magnet fixed on each tool handle, and two poles of the fixing block permanent magnet are arranged to be in an inverse relationship with two poles of the tool handle permanent magnet.

An S pole and an N pole of the fixing block permanent magnet are fixed on any end surface of the fixing block in an up-down relative relationship, and an S pole and an N pole of the permanent magnet on the tool handle are arranged in an opposite direction to the magnetic poles of the permanent magnet on the fixing block according to the principle that heteropoles attract.

The permanent magnets of the two tool handles may be disposed on ends of the connecting shafts, i.e. that they may be disposed on ends of the connecting shafts adjacent to the handle actuators or ends of the connecting shafts adjacent to the urinary tract actuators.

The tool handle permanent magnets and the fixing block permanent magnet are disposed in the same plane, wherein the tool handle permanent magnets are positioned on two sides, while the fixing block permanent magnet is positioned between them.

Preferably, the connecting portion is in the form of a connecting strip. The connecting strip is provided with a pin shaft mounting lug, and the pin shaft mounting lug is provided with a mounting hole; the fixing block is a pin shaft passing through the mounting holes of the pin shaft mounting lugs of the two tool handles; the two tool handles form scissor-type rotational connection via the pin shaft,
i.e. that the two tool handles are rotationally connected via the pin shaft.

Preferably, the tool handle rotation angle limiting mechanisms each comprise a torsion spring. The torsion spring is sleeved on the pin shaft, and spring ends respectively abut against the connecting strips of the two tool handles, such that the two tool handles form an elastically repeatable scissor-type rotation around the pin shaft.

The spring sheet described herein is specifically embodied in the form of a torsion spring so as to achieve the function of a spring and adapt to the mounting structure.

Preferably, the two tool handles are of a planarly symmetric spatial shape.

A planarly symmetric shape means that along the middle line between two connecting shafts, the connecting shafts and the handle actuators are mirror-symmetric. Although the urinary tract actuators are arranged in a front-and-back staggered manner along the direction of the urinary tract, they are also mirror-symmetric in shape along the middle vertical plane between the two tool handles.

Here, the connecting shaft should be construed broadly. In other words, it can be considered that connection can also be achieved by means of the connecting portion, and the effects achieved after actual molding are also similar to those achieved by directly adopting the narrowly construed connecting shaft.

The handle actuators of the two tool handles are positioned in the same plane, while the urinary tract actuators of the two tool handles are positioned in different planes.

Preferably, the plane where the handle actuators of the two tool handles are positioned forms an included angle of from 90 to 120 degrees with respect to the plane where the connecting shafts of the two tool handles are positioned.

Here, the connecting shaft can generally be construed broadly. In other words, it is considered that connection can also be achieved by means of its broadly construed connecting portion, and the functional effects achieved after actual molding of the connecting portion are also similar to those achieved by directly adopting the narrowly construed connecting shaft. Nonetheless, the structure is relatively stable when the narrowly construed connecting shaft is adopted for connection.

Preferably, the plane where the handle actuators of the two tool handles are positioned forms an included angle of from 90 to 120 degrees with respect to the plane where the connecting portions of the two tool handles are positioned.

Further, the plane where the tool handle actuators are positioned forms an included angle of 100 degrees with respect to the plane where the connecting shafts of the two tool handles are positioned, which can achieve the optimal hand-holding effects.

The handle actuators of the two tool handles are arranged in the same plane, but the urinary tract actuators must not be in the same plane so as to avoid such complications as urinary tract necrosis caused by their influence on the supply of blood to the urinary tract.

The urinary tract actuators of the tool handles are arc-shaped structures extending towards lateral sides, tail ends of which are buckled inwards.

Each urinary tract actuator is in the shape of a semi-elliptical arc.

Such a shape is designed to provide better control of the urinary tract. The cross-sectional shape of the urinary tract is substantially circular, which becomes substantially elliptical after the urinary tract is pressed. Moreover, two urinary tract actuators just form a substantially elliptical shape after pressing the urinary tract.

Preferably, the tool handles each comprise one or more urinary tract actuators. The urinary tract actuators of the two tool handles are arranged in a mutually staggered manner,
thereby avoiding local stress and reducing tissue necrosis.

Preferably, one of the tool handles is provided with a single urinary tract actuator, the other tool handle is provided with two urinary tract actuators, and the single urinary tract actuator is inserted between the two urinary tract actuators.

As such, the maximum balance between miniaturization and reduction of local stress can be achieved, and the ease of use of the equipment is maximized.

In the present invention, the urinary tract actuators are oppositely arranged on two sides of the urinary tract and act to squeeze the urinary tract. They may be implanted within a penis via surgical procedures. In other words, these urinary tract actuators may be implanted between the urinary tract and the cavernous body within a penis via surgical procedures. Moreover, the handle actuators are positioned on two sides of the cavernous body within the penis.

The present invention provides an implantable urination controlling tool that can be implanted between a urinary tract and a corpus cavernosum of a male. The implantable urination controlling tool is composed of two symmetrically arranged tool handles and a fixing block used for limiting the axial or cross rotation of the tool handles. Here, the two tool handles are of a planarly symmetric shape. When implanted into a penis, the two tool handles are respectively arranged on two sides of the cavernous body of the penis. The tool handles are provided with such structures as urinary tract actuators, connecting portions, tool handle rotation angle limiting mechanisms and handle actuators. The urinary tract actuators are used for squeezing the urinary tract, and the urinary tract actuators on the two tool handles are arranged in a front-and-back crossed manner along the direction of the urinary tract, i.e. that the urinary tract is clamped between the urinary tract actuators. Moreover, the urinary tract actuators do not clamp at the same point on the urinary tract; rather, they are always in a state in which they are staggered in a front-and-back manner. The connecting portions are used for connecting the urinary tract actuators and the handle actuators. The connecting portions of the two tool handles are different in length, thereby ensuring that the two urinary tract actuators do not clamp at the same point on the urinary tract when the handle actuators are mounted in the same plane. The fixing block is positioned between the cavernous body and the urinary tract of the penis, and may be provided with grooves. The tool handle rotation angle limiting mechanisms are used for limiting ranges of positive and negative included angles corresponding to the opening and closing actions that can be achieved by the two tool handles when rotating. The rotation angle limiting mechanisms may be positioned on the middle segments or ends of the connecting shafts, or abut against side surfaces of the connecting portions. Meanwhile, the tool handle rotation angle limiting mechanisms may each comprise a spring sheet mounted on the fixing block and a torque increasing structure interacting with the spring sheet. The tool handle rotation angle limiting mechanisms may also control the opening and closing of the urinary tract by means of a permanent magnet structure, and such forms as electromagnets may be used if necessary. Meanwhile, spacing protrusions may also be used so as to avoid the failure of other rotation angle limiting mechanisms.

The present invention has the following beneficial effects: The implantable urination controlling tool provided by the present invention is simple and stable in structure, less in trauma induced by the implantation manner and ideal in urinary control. Moreover, this tool not only well solves the problem of urinary incontinence; more importantly, no trace of it can be found from the body surface, and it can be used conveniently in daily life and is maintenance-free for life.

### BRIEF DESCRIPTION OF THE DRAWINGS

Figure 1 is a structural schematic view of a first embodiment of the present invention;
Figure 2 is an exploded view of the first embodiment of the present invention;
Figure 3 is a schematic view of a fixing block in the first embodiment of the present invention;
Figure 4 is an exploded view of a second embodiment of the present invention;
Figure 5 is a structural schematic view of the second embodiment of the present invention;
Figure 6 is a schematic view of a fixing block in the second embodiment of the present invention;
Figure 7 is a side view of the second embodiment of the present invention;
Figure 8 is a cross-sectional view taken along line A-A of Figure 7;
Figure 9 is a structural schematic view of a third embodiment of the present invention;
Figure 10 is an exploded view of the third embodiment of the present invention;
Figure 11 is a schematic view of a fixing block in the third embodiment of the present invention;
Figure 12 is a structural schematic view of a fourth embodiment of the present invention;
Figure 13 is an exploded view of the fourth embodiment of the present invention;
Figure 14 is a schematic view of a fixing block in the fourth embodiment of the present invention;
Figure 15 is a front view of the fixing block in the fourth embodiment of the present invention;
Figure 16 is a schematic view of a tool handle in the fourth embodiment of the present invention;
Figure 17 is a structural schematic view of a fifth embodiment of the present invention; and
Figure 18 is an exploded view of the fifth embodiment of the present invention.

In the figures, various components and reference numerals thereof are illustrated as follows:
1-tool handle; 1.1-handle actuator; 1.2a-connecting shaft;
1.2b-connecting strip; 1.21b-pin shaft mounting lug; 1.211b-mounting hole;
1.2.1-spring contact surface; 1.2.2-connecting segment; 1.3-urinary tract actuator;
2-fixing block; 2.1-communication hole; 2.2-avoidance groove;
2.3-mounting hole; 2.4-window; 2.5-pin shaft;
3-tool handle rotation angle limiting mechanism;
3.1-compressible spring; 3.1.1-pin member; 3.2-fixing block permanent magnet;
3.3-tool handle permanent magnet; 3.4-torsion spring; 3.5-spacing protrusion.

### DETAILED DESCRIPTION

The present invention is further illustrated below in conjunction with the accompanying drawings and specific embodiments.

### Embodiment 1

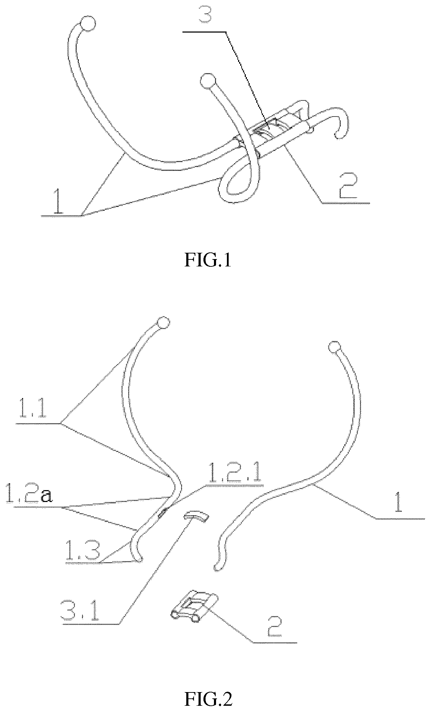

Illustrated in Figures 1-3 is an implantable urination controlling tool, which is composed of two tool handles 1, a fixing block 2 and tool handle rotation angle limiting mechanisms 3. The two tool handles 1 are, respectively, a first tool handle and a second tool handle. The tool handles 1 are each composed of a handle actuator 1.1, a connecting shaft 1.2a and a urinary tract actuator 1.3. The handle actuator 1.1 extends from an end of the connecting shaft 1.2a in an upward bending manner, and the urinary tract actuator 1.3 extends from the other end of the connecting shaft in a downward bending manner. The first tool handle and the second tool handle are arranged to be mirror-symmetric with respect to each other, and in particular, parts of the handle actuators 1.1 and the connecting shafts 1.2a are symmetric with respect to the central axis. The handle actuators 1.1 are arc-shaped structures extending towards lateral sides, terminal ends of which are bent inwards. The included angle between the plane formed by two handle actuators 1.1 and the plane formed by two connecting shafts 1.2a is an obtuse angle of 100 degrees. The handle actuators 1.1 may be toggled inwards to make the connecting shafts 1.2a rotate inwards. The word "Inwards" described herein refers to a direction between the two connecting shafts 1.2a, while the word "Outwards" corresponds to lateral sides of the connecting shafts 1.2a. The connecting shafts 1.2a of the two tool handles 1 are parallelly sleeved in communication holes 2.1 within the fixing block 2. The urinary tract actuators 1.3 of the tool handles 1 are also arc-shaped structures bent towards lateral sides, terminal ends of which are bent inwards for clamping. Specifically, they are arc-shaped structures having a substantially semi-elliptical shape, thereby conforming to the shape of the urinary tract. However, the urinary tract actuator of the first tool handle is positioned in front of the urinary tract actuator of the second tool handle. It is specified herein that along the direction of the urinary tract, the direction along which urine flows out is referred to as a front position, while the direction along which urine flows in is referred to as a back position. With regard to the present device, it can be considered that based on the connecting shaft 1.2a, the direction along which the axis of the urinary tract actuator 1.3 extends is referred to as a front position, while the direction along which the axis of the handle actuator 1.1 extends is referred to as a back position. The plane where the urinary tract actuator 1.3 of the first tool handle is positioned is different from, but parallel to, the plane where the urinary tract actuator 1.3 of the second tool handle is positioned. Moreover, the two urinary tract actuators are still symmetric in shape, but staggered with respect to each other. This solution is implanted on the urinary tract below the pubis of a male. The urinary tract actuator faces into the human body, the handle actuator faces towards the body surface of the human body, and the included angle between the handle actuator and the connecting shaft is in a range of from 90 to 120 degrees, and preferably, 100 degrees. Such a shape is designed to accommodate the physiological structure of the penis of a male.

The urinary tract actuators 1.3 are used for squeezing the urinary tract, and the urinary tract actuators 1.3 on the two tool handles 1 are arranged in a front-and-back crossed manner along the direction of the urinary tract, i.e. that the urinary tract is clamped between two urinary tract actuators 1.3. Moreover, the two urinary tract actuators 1.3 do not clamp at the same point on the urinary tract; rather, they are always in a state in which they are staggered in a front-and-back manner. It may also be considered that the connecting shafts 1.2a of the two tool handles 1 are different in length, thereby ensuring that the two urinary tract actuators 1.3 do not clamp at the same point on the urinary tract when the handle actuators 1.1 are mounted in the same plane. The middle segments of the connecting shafts 1.2a of the two tool handles are each provided with a clamping slot, the inner surface of which is the spring contact surface 1.2.1. A window 2.4 is disposed in the middle of the fixing block 2, and inner walls on two sides of the window 2.4 are provided with grooves 2.2 in communication with the communication holes 2.1. It may be considered that the grooves 2.2 are positioned on inner sides of the fixing block 2, and the window 2.4 is used for providing a motion space for the tool handles 1 to rotate. The tool handle rotation angle limiting mechanisms 3 are compressible springs 3.1, and two ends of the compressible springs 3.1 are connected with the clamping slots on the connecting shafts 1.2a in a mutually clamping manner after passing through the grooves 2.2. The compressible springs 3.1 are used for limiting the range of positive and negative included angles corresponding to the opening and closing actions that can be achieved by the two tool handles 1 when rotating. In other words, when the tool handles 1 rotate, the spring can only be stabilized in the state in which the urinary tract actuators squeeze to block the urinary tract, while other states all are process states, namely, non-stable states. When the compressible spring 3.1 is in a preset pressure state, namely in an initial state, the two urinary tract actuators 1.3 are in a clamping state in which they are staggered in a front-and-back manner. When toggled inwards, the two handle actuators 1.1 move in a relative direction. Driven by the handle actuators, the two connecting shafts 1.2a rotate inwards, such that the two urinary tract actuators 1.3 rotate oppositely to release the urinary tract. At this time, the compressible spring 3.1 is stressed to deform, thereby producing a restoring force for closing the urinary tract. Specifically, when a patient needs to urinate, the urinary tract can be opened for urination simply by manually moving the two urinary tract actuators inwards. When urination is over, as the compressible spring has a restoring force for closing the urinary tract, all that is needed is for the patient to release his hands, and then, under the action of such a restoring force, the two urinary tract actuators will return to the stable state in which the urinary tract is occluded.

### Embodiment 2

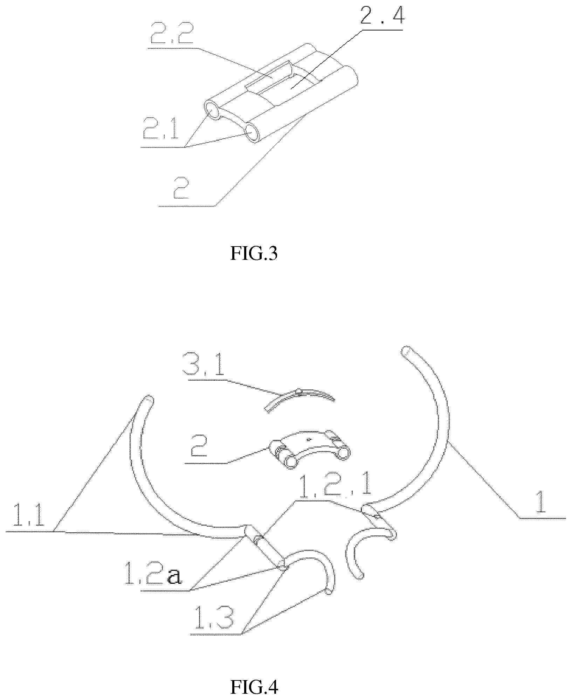

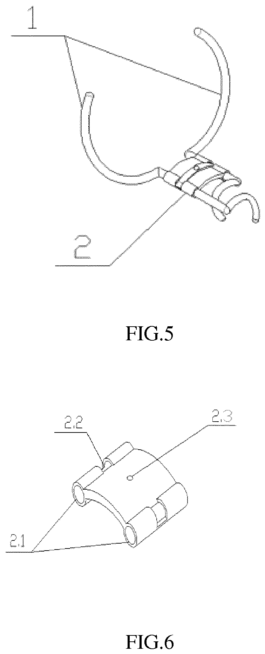

Illustrated in Figures 4-8 is an implantable urination controlling tool, the remaining features of which are the same as those described in Embodiment 1. The first tool handle and the second tool handle are arranged to be mirror-symmetric with respect to each other, and in particular, parts of the handle actuators 1.1 and the connecting shafts 1.2a are symmetric with respect to the central plane. The handle actuators 1.1 are arc-shaped structures extending towards lateral sides, terminal ends of which are bent inwards. The included angle between the plane formed by two handle actuators 1.1 and the plane formed by two connecting shafts 1.2a is an obtuse angle of 110 degrees. The handle actuators 1.1 may be toggled inwards to make the connecting shafts 1.2a rotate inwards. The connecting shafts 1.2a of the two tool handles 1 are parallelly sleeved in communication holes 2.1 within the fixing block 2. The urinary tract actuators 1.3 of the tool handles 1 are arc-shaped structures bent towards opposite lateral sides. The two urinary tract actuators 1.3 are arranged in a front-and-back crossed manner, terminal ends of which are bent inwards for clamping. However, the urinary tract actuator of the first tool handle is positioned in front of the urinary tract actuator of the second tool handle. Outer sides of the middle segments of the connecting shafts 1.2a of the two tool handles are spring contact surfaces 1.2.1. Grooves 2.2 are disposed in upper portions of the communication holes 2.1 on two sides of the fixing block 2, and the spring contact surfaces 1.2.1 on the middle segments of the connecting shafts 1.2a can be seen through the grooves 2.2. The middle portion of the fixing block 2 is provided on an upper surface thereof with a mounting hole 2.3. The tool handle rotation angle limiting mechanisms 3 are compressible springs 3.1. The compressible springs 3.1 are arched, two ends of which abut the spring contact surfaces 1.2.1. The compressible springs 3.1 are provided in the middle portions thereof with a pin member 3.1.1 for fixedly connecting with the mounting hole 2.3. The compressible springs 3.1 are used for limiting ranges of positive and negative included angles corresponding to the opening and closing actions that can be achieved by the two tool handles 1 when rotating. In other words, when the tool handles 1 rotate, the springs can only be stabilized in the state in which the urinary tract actuators squeeze to block the urinary tract, while other states all are process states, namely, non-stable states. When the compressible springs 3.1 are in a preset pressure state, namely in an initial state, the two urinary tract actuators 1.3 are in a clamping state in which they are staggered in a front-and-back manner. When toggled outwards, the two handle actuators 1.1 move in opposite directions. Driven by the handle actuators, the two connecting shafts 1.2a rotate in opposite directions, such that the two urinary tract actuators 1.3 rotate in opposite directions to release the urinary tract. At this time, the compressible spring 3.1 is stressed to deform, thereby producing a restoring force for closing the urinary tract. Specifically, when a patient needs to urinate, the urinary tract can be opened for urination simply by manually moving the two urinary tract actuators outwards. When urination is over, as the compressible spring has a restoring force for closing the urinary tract, all that is needed is for the patient to release his hands, and then, under the action of such a restoring force, the two urinary tract actuators will return to the stable state in which the urinary tract is occluded. This arrangement serves as a supplementary solution. It may be pinched inwards to function as opening control for the urinary tract, just as the solution described in Embodiment 1; in addition, it may also be manually moved outwards to function as the opening control, just as the solution described in Embodiment 2.

### Embodiment 3

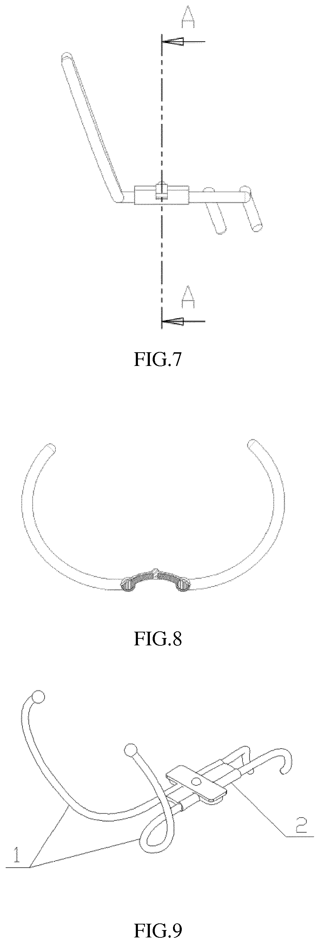

Illustrated in Figures 9-11 is an implantable urination controlling tool, the remaining features of which are the same as those described in Embodiment 2. The first tool handle and the second tool handle are arranged to be mirror-symmetric with respect to each other, and in particular, parts of the handle actuators 1.1 and the connecting shafts 1.2a are symmetric with respect to the central axis. The handle actuators 1.1 are arc-shaped structures extending towards lateral sides, terminal ends of which are bent inwards. The included angle between the plane formed by two handle actuators 1.1 and the plane formed by two connecting shafts 1.2a is an obtuse angle of 120 degrees. The handle actuators 1.1 may be toggled inwards to make the connecting shafts 1.2a rotate inwards. The connecting shafts 1.2a of the two tool handles 1 are parallelly sleeved in communication holes 2.1 within the fixing block 2. The urinary tract actuators 1.3 of the tool handles 1 are also arc-shaped structures bent towards opposite lateral sides, terminal ends of which are bent inwards for clamping. However, the urinary tract actuator of the first tool handle is positioned in front of the urinary tract actuator of the second tool handle. The middle segments of the connecting shafts 1.2a of the two tool handles are each provided with a connecting segment 1.2.2 shaped like a Chinese character "Ji". The connecting segment 1.2.2 protrudes outwards, the upper surface of which is a spring contact surface 1.2.1. Grooves 2.2 are disposed in upper portions and lateral sides of the communication holes 2.1 on two sides of the fixing block 2, and the connecting segments 1.2.2 extend outwards from the grooves 2.2. The upper portion in the middle of the fixing block 2 is provided with a mounting hole 2.3. The tool handle rotation angle limiting mechanisms 3 are compressible springs 3.1. The compressible springs 3.1 are plate-shaped, two ends of which abut the spring contact surfaces. The compressible springs 3.1 are provided in the middle portions thereof with a pin member 3.1.1 for fixedly connecting with the mounting hole 2.3. The compressible spring 3.1 are used for limiting ranges of positive and negative included angles corresponding to the opening and closing actions that can be achieved by the two tool handles 1 when rotating. In other words, when the tool handles 1 rotate, the spring can only be stabilized in the state in which the urinary tract actuators squeeze to block the urinary tract, while other states all are process states, namely, non-stable states. When the compressible spring 3.1 is in a preset pressure state, namely in an initial state, the two urinary tract actuators 1.3 are in a clamping state in which they are staggered in a front-and-back manner. When toggled inwards, the two handle actuators 1.1 move in a relative direction. Driven by the handle actuators, the two connecting shafts 1.2a rotate inwards, such that the two urinary tract actuators 1.3 rotate oppositely to release the urinary tract. At this time, the compressible spring 3.1 is stressed to deform, thereby producing a restoring force for closing the urinary tract. Specifically, when a patient needs to urinate, the urinary tract can be opened for urination simply by manually moving the two urinary tract actuators inwards. When urination is over, as the compressible spring has a restoring force for closing the urinary tract, all that is needed is for the patient to release his hands, and then, under the action of such a restoring force, the two urinary tract actuators will return to the stable state in which the urinary tract is occluded.

### Embodiment 4

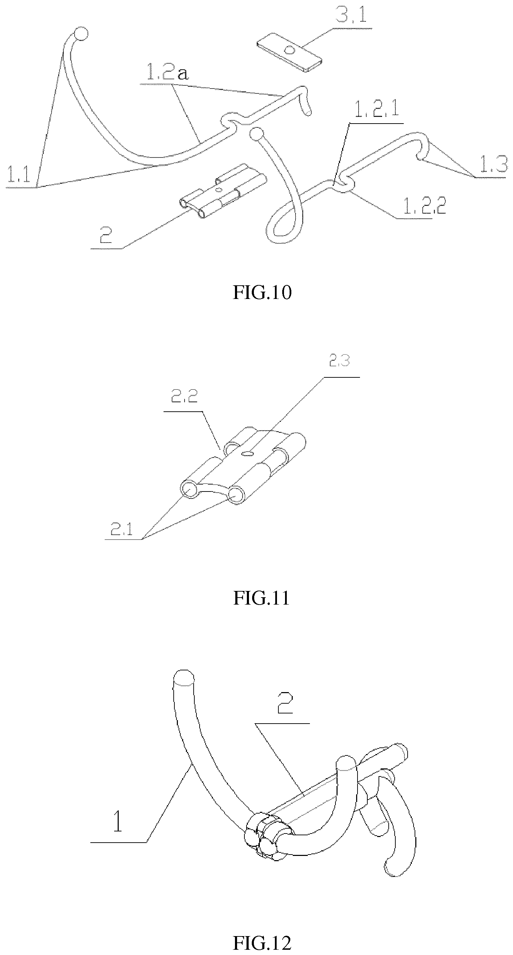

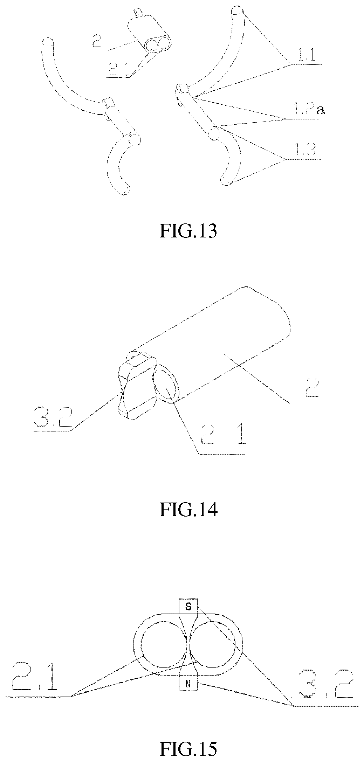

Illustrated in Figures 12-16 is an implantable urination controlling tool. The tool handles 1 are each composed of a handle actuator 1.1, a connecting shaft 1.2a and a urinary tract actuator 1.3. The handle actuator 1.1 extends from an end of the connecting shaft 1.2a in an upward bending manner, and the urinary tract actuator 1.3 extends from the other end of the connecting shaft in a downward bending manner. The first tool handle and the second tool handle are arranged to be mirror-symmetric with respect to each other, and in particular, parts of the handle actuators 1.1 and the connecting shafts 1.2a are symmetric with respect to the central axis. The included angle between the plane formed by two handle actuators 1.1 and the plane formed by two connecting shafts 1.2a is a right angle of 90 degrees. The handle actuators 1.1 may be toggled inwards to make the connecting shafts 1.2a rotate inwards. The handle actuators 1.1 may be toggled inwards to make the connecting shafts 1.2a rotate inwards The connecting shafts 1.2a of the two tool handles 1 are parallelly sleeved in communication holes 2.1 within a fixing block 2. The urinary tract actuators 1.3 of the tool handles 1 are also arc-shaped structures bent towards opposite lateral sides, terminal ends of which are bent inwards for clamping. However, the urinary tract actuator of the first tool handle is positioned in front of the urinary tract actuator of the second tool handle. The urinary tract actuators 1.3 are used for squeezing the urinary tract, and the urinary tract actuators on the two tool handles are arranged in a front-and-back crossed manner along the direction of the urinary tract, i.e. that the urinary tract is clamped between two urinary tract actuators. Moreover, the two urinary tract actuators do not clamp at the same point on the urinary tract; rather, they are always in a state in which they are staggered in a front-and-back manner. It may also be considered that the connecting shafts 1.2a of the two tool handles are different in length, thereby ensuring that the two urinary tract actuators 1.3 do not clamp at the same point on the urinary tract when the handle actuators 1.1 are mounted in the same plane. The tool handle rotation angle limiting mechanisms 3 are permanent magnet assemblies, which each comprise a fixing block permanent magnet 3.2 and a tool handle permanent magnet 3.3. The tool handle rotation angle limiting mechanisms 3 each composed of the tool handle permanent magnet 3.3 and the fixing block permanent magnet 3.2 is used for limiting the range of positive and negative included angles corresponding to the opening and closing actions that can be achieved by the two tool handles when rotating. In other words, when the tool handles 1 rotate, the mechanism can only be stabilized in the state in which the urinary tract actuators squeeze to block the urinary tract and the state in which the urinary tract actuators release to open the urinary tract, while other states all are temporary. During this process, a stable state can be reached only when the tool handle permanent magnet 3.3 and the fixing block permanent magnet 3.2 fully abut together. The fixing block permanent magnet 3.2 is disposed on an end of the fixing block 2, and its S pole and N pole are disposed above and below the fixing block 2, respectively. Moreover, the tool handle permanent magnet 3.3 is disposed on the connecting shaft 1.2a corresponding to the end of the fixing block 2. The tool handle permanent magnet on the connecting shaft 1.2a is oppositely arranged, i.e. that the N pole is disposed above, while the S pole is disposed below. The connecting shafts 1.2a of the two tool handles 1 are each provided with the tool handle permanent magnet 3.3, and the three permanent magnets may attract one another directly. The permanent magnet attraction above serves as a closing stable state, while the permanent magnet attraction below serves as an opening stable state. The handle actuators may be toggled to make the urinary tract actuators rotate outwards to open the urinary tract. When urination is over, the handle actuators may then be toggled inwards to make the urinary tract actuators rotate inwards to close the urinary tract.

### Embodiment 5

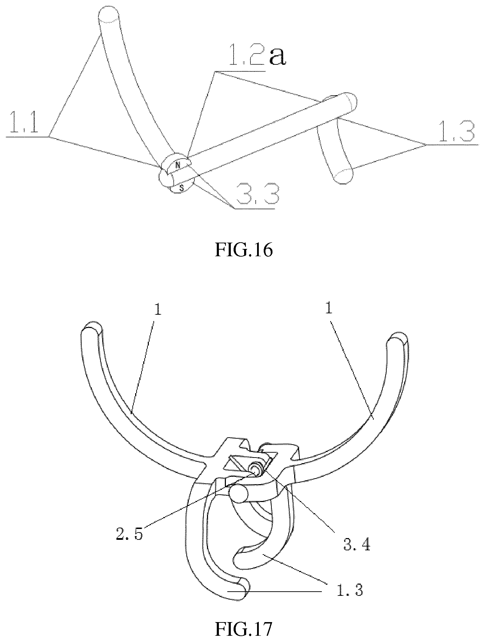

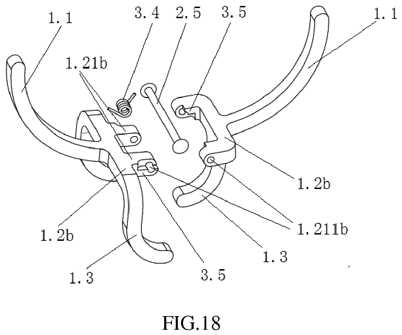

Illustrated in Figures 17 and 18 is an implantable urination controlling tool, which is composed of two tool handles 1, a pin shaft 2.5 and a torsion spring 3.4. The tool handles 1 each comprise a handle actuator 1.1, a connecting strip 1.2b and a urinary tract actuator 1.3. The handle actuator 1.1 is an arc-shaped structure, an end of which is connected to the middle portion of the connecting strip 1.2b, and the other end is bent inwards. Two handle actuators 1.1 are arranged to be mirror-symmetric with respect to each other, and the plane formed by them is perpendicular to the connecting strip 1.2b. The urinary tract actuator 1.3 is an arc-shaped structure having a distal end bent inwards. One of the tool handles 1 is provided with two urinary tract actuators 1.3, which are arranged at two ends of the connecting strip 1.2b corresponding to the handle actuator 1.1. The other tool handle 1 is provided with one urinary tract actuator 1.3, which is arranged in the middle of the connecting strip 1.2b corresponding to the handle actuator 1.1. The inwards-bent structures of the urinary tract actuators 1.3 all are arranged inwards in a face-to-face manner for clamping the urinary tract. Moreover, the urinary tract actuator 1.3 on one side is positioned between two urinary tract actuators 1.3 on the other side, and the three are arranged in a staggered manner to clamp the urinary tract. The two connecting strips 1.2b are provided respectively with a pin shaft mounting lug 1.21b. The pin shaft mounting lugs 1.21b are provided with mounting holes 1.211b having an identical diameter, and the pin shaft 2.5 rotationally connects the two connecting strips 1.2b through the mounting holes 1.211b. When the relative rotation of the two tool handles 1 reaches a set angle, spacing protrusions 3.5 arranged on one connecting strip 1.2b will come into contact with spacing protrusions 3.5 arranged on the opposite connecting strip 1.2b so as to prevent the two tool handles 1 from continuing to rotate, thereby reaching the purpose of spacing. The torsion spring 3.4 is arranged on the pin shaft 2.5. One of its leads is placed on the connecting strip 1.2b on one side, and the other one of its leads is placed on the connecting strip 1.2b on the other side. The action force of the torsion spring 3.4 maintains the two tool handles 1 in a stable state in which the urinary tract is occluded, namely a state in which the urinary tract actuators 1.3 approach one another to clamp the urinary tract. When urination is required, an external force may be applied to make the handle actuators 1.1 approach each other in an inward direction. At this time, the torsion spring 3.4 is compressed, and the urinary tract actuators 1.3 move away from one another, thereby releasing the urinary tract and rendering it in a urination state.

The preferred embodiments of this invention-creation have been described above in detail, but this invention-creation is not limited to the described embodiments. Without departing from the spirit of this invention-creation, those skilled in the art may make various equivalent modifications or substitutions that are covered by the scope defined in the claims of the present application.

## Claims

1. An implantable urinary control device, comprising two tool handles, wherein each of the tool handles comprises a connecting portion, a urinary tract actuator and a handle actuator; the implanted urinary control device also comprises a fixing block for connecting with the connecting portions of the two tool handles; the urinary tract actuator of one of the tool handles is positioned in front of the urinary tract actuator of the other tool handle; and the implantable urinary control device further comprises tool handle rotation angle limiting mechanisms for limiting opening angles of the urinary tract actuators.

2. The implantable urinary control device of claim 1, wherein the connecting portions of the two tool handles are parallel to each other, and the tool handle rotation angle limiting mechanism is disposed on the connecting portions.

3. The implantable urinary control device of claim 2, wherein the tool handle rotation angle limiting mechanism is a spacing protrusion; the spacing protrusion is disposed on the connecting portions; when one of the tool handles rotates, its spacing protrusion abuts against the spacing protrusion of the other tool handle upon rotation, thereby preventing the tool handles from continuing to rotate.

4. The implantable urinary control device of claim 2, wherein the tool handle rotation angle limiting mechanisms are spring sheets or permanent magnets.

5. The implantable urinary control device of claim 4, wherein the tool handle rotation angle limiting mechanism further comprises a spacing protrusion; the spacing protrusion is disposed on the connecting portion; when one of the tool handles rotates, its spacing protrusion abuts against the spacing protrusion of the other tool handle upon rotation, thereby preventing the tool handles from continuing to rotate.

6. The implantable urinary control device of any one of claim 4 or 5, wherein the connecting portion is in the form of a connecting shaft.

7. The implantable urinary control device of claim 6, wherein the tool handle rotation angle limiting mechanisms are compressible springs, wherein the connecting shafts are provided with spring contact surfaces; two ends of the compressible springs come into contact with the spring contact surfaces of the two connecting shafts, respectively; and the fixing block is provided with grooves for avoiding the compressible springs from coming into contact with the spring contact surfaces.

8. The implantable urinary control device of claim 7, wherein a window is disposed in the middle portion of the fixing block, wherein the grooves are positioned on side walls of the window and are also in communication with communication holes of the connecting shafts; the spring contact surface on each of the connecting shafts is a clamping slot; and two ends of the compressible spring respectively passes through the grooves of the fixing block to be connected with the clamping slots on the connecting shafts in a clamping manner.

9. The implantable urinary control device of claim 7, wherein the grooves are positioned on two sides of the fixing block and are also in communication with the communication holes of the connecting shafts, wherein the fixing block is provided centrally with a mounting hole for fixedly connecting with a pin member in the center of the spring sheet, and two ends of the spring sheet abut the connecting shafts of the two tool handles.

10. The implantable urinary control device of claim 8, wherein the spring sheet is arched, and inner sides of the two ends of the spring sheet abut the connecting shafts of the two tool handles.

11. The implantable urinary control device of claim 8, wherein the spring sheet is planar, wherein the connecting shafts of the tool handles extend out of the grooves to form connecting segments, and two ends of the planar spring sheet abut the connecting segments.

12. The implantable urinary control device of claim 6, wherein the tool handle rotation angle limiting mechanism is composed of a permanent magnet fixed on the fixing block and a permanent magnet fixed on each tool handle, wherein two poles of the fixing block permanent magnet are arranged to be in an inverse relationship with two poles of the tool handle permanent magnet.

13. The implantable urinary control device of any one of claims 3-5, wherein the connecting portion is in the form of a connecting strip, wherein the connecting strip is provided with a pin shaft mounting lug, and the pin shaft mounting lug is provided with a mounting hole; the fixing block is a pin shaft passing through the mounting holes of the pin shaft mounting lugs of the two tool handles; and the two tool handles form scissor-type rotational connection via the pin shaft.

14. The implantable urinary control device of claim 13, wherein the tool handle rotation angle limiting mechanism comprises a torsion spring, wherein the torsion spring is sleeved on the pin shaft, and spring ends respectively abut against the connecting strips of the two tool handles, such that the two tool handles form an elastically repeatable scissor-type rotation around the pin shaft.

15. The implantable urinary control device of any one of claims 6-14, wherein the two tool handles are of a planarly symmetric spatial shape.

16. The implantable urinary control device of claim 15, wherein the handle actuators of the two tool handles are positioned in the same plane, while the urinary tract actuators of the two tool handles are positioned in different planes.

17. The implantable urinary control device of any one of claims 1-16, wherein the plane where the handle actuators of the two tool handles are positioned forms an included angle of from 90 to 120 degrees with respect to the plane where the connecting portions of the two tool handles are positioned.

18. The implantable urinary control device of claim 16, wherein the plane where the handle actuators of the two tool handles are positioned forms an included angle of from 90 to 120 degrees with respect to the plane where the connecting shafts of the two tool handles are positioned.

19. The implantable urinary control device of claim 16, wherein each urinary tract actuator is an arc-shaped structure extending towards a lateral side and having a tail end buckled inwards.

20. The implantable urinary control device of any one of claims 1-19, wherein the tool handles each comprises one or more urinary tract actuators, wherein the urinary tract actuators of the two tool handles are arranged in a mutually staggered manner.

21. The implantable urinary control device of claim 20, wherein one of the tool handles is provided with a single urinary tract actuator, the other tool handle is provided with two urinary tract actuators, and the single urinary tract actuator is inserted between the two urinary tract actuators.
